# EUROPÄISCHE PATENTSCHRIFT

(11) **EP 0 891 745 B1**
(45) Veröffentlichungstag und Bekanntmachung des Hinweises auf die Patenterteilung: **11.12.2002**
(21) Anmeldenummer: 98109027.7
(22) Anmeldetag: 18.05.1998
(51) Int. Cl.: A61B 18/00

(54) **Elektrisch betriebene medizinische Vorrichtung**
Electrically operated medical device
Appareil médical à énergie électrique

(30) Priorität: 16.07.1997 DE 19730456
(43) Veröffentlichungstag der Anmeldung: 20.01.1999
(73) Patentinhaber: Berchtold Holding GmbH, 78532 Tuttlingen (DE)
(72) Erfinder: Tockweiler, Udo, 78194 Immendingen (DE); Schilling, Bertram, 78194 Immendingen 3 - Mauenheim (DE)
(74) Vertreter: Manitz, Gerhart, Dr.

(56) Entgegenhaltungen:
- EP-A- 0 424 687
- EP-A- 0 424 792
- EP-A- 0 525 539
- US-A- 4 696 309
- US-A- 5 561 699

## Beschreibung

Die Erfindung betrifft eine elektrisch betriebene medizinische Vorrichtung, wie eine elektrisch-chirurgische Vorrichtung, nach dem Oberbegriff des Patentanspruches 1.

In Operationssälen werden viele elektrisch betriebene Vorrichtungen verwendet, bei denen zwischen Patient und Vorrichtung elektrische Leitungen oder Schlauchverbindungen vorliegen müssen. Da um den Operationstisch herum während der Operation immer Platzprobleme bestehen, stören derartige Leitungen und Verbindungen erheblich. Besonders kritisch sind hierbei Verbindungsleitungen, die am Boden entlang unter dem Operationstisch verlegt sind, wie z.B. Zuleitungen zu Fußschaltern, über die der Chirurg Gerätefunktionen z.B. innerhalb eines Hochfrequenz-Chirurgiegerätes ansteuern kann. Die Fußbedienung ist deswegen oft notwendig, da der Chirurg beide Hände für das Manipulieren mit den Instrumenten benötigt, von denen mindestens eines an das elektrische Speise- und Steuergerät angeschlossen ist. Da die elektrisch betriebenen medizinischen Vorrichtungen aus Platzgründen meist in einer gewissen Entfernung vom Operationstisch untergebracht sind, müssen die Leitungen am Fußboden relativ lang sein, so daß sie die freie Bewegung der um den Operationstisch herumstehenden Personen behindern.

Aus der EP 0 525 539 A2 ist bereits eine zahnärztliche Behandiungseinrichtung bekannt, wobei Steuersignale von einem Fußkontakt an eine zentrale Steuereinheit übertragen werden. Um die Störanfälligkeit zu verringern und eine besonders einfache Bedienung zu ermöglichen, ist vorgesehen, daß eine Druckbelastung einer Oberfläche des Fußkontakts in primäre elektrische Signale umgewandelt wird und daß die primären Signale in Steuersignale für die zentrale Steuereinheit umgesetzt werden.

Das Ziel der Erfindung besteht darin, eine elektrisch betriebene medizinische Vorrichtung der eingangs genannten Gattung so auszubilden, daß nicht nur der Aufwand für die Leitungsverlegung minimiert wird, so daß insbesondere im kritischen Bereich unterhalb des Operationstisches und um diesen herum Störungen durch dort verlegte Leitungen weitgehend unterbunden sind, sondern auch auf einfache Weise eine Auswechslung des erfindungsgemäßen Fußschalters gegen einen herkömmlichen Fußschalter möglich ist.

Zur Lösung dieser Aufgabe sind die Merkmale des kennzeichnenden Teils des Patentanspruches 1 vorgesehen.

Erfindungsgemäß wird also nicht nur auf Leitungsverbindungen zwischen den insbesondere als Fußschalter ausgebildeten Schalter und einer vorzugsweise nahe dem Speise- und Steuergerät angeordneten Empfangsstation völlig verzichtet, so daß die diesbezüglichen Behinderungen der am Operationstisch arbeitenden Personen völlig vermieden sind, sondern es kann ein herkömmlicher Fußschalter einfach durch einen erfindungsgemäßen ausgetauscht werden, indem der Steckkontakt bzw. die Steckbuchse des herkömmlichen Fußschalters aus dem Speise- und Steuergerät abgezogen und durch den Steckkontakt bzw. die Steckbuchse der Empfangsstation oder einer Verbindung eines erfindungsgemäßen Fußschalters ersetzt wird. Auf diese Weise ist die erfindungsgemäße elektrisch betriebene medizinische Vorrichtung in besonders universeller Weise verwendbar.

Vorteilhafte Weiterbildungen der Erfindung entnimmt man den Ansprüchen 2 und 3.

Ein besonderes Problem bei elektrisch betriebenen medizinischen Vorrichtungen besteht darin, daß die Störsicherheit der Übertragung sehr hoch sein muß. Dies ist in Operationssälen besonders schwierig zu realisieren, da dort zahlreiche potentielle Störvorrichtungen, wie Hochfrequenz-Chirurgiegeräte, Röntgengeräte, NMR-Tomographen oder ähnliche komplizierte elektronische Geräte vorhanden sein können, gegen die eine absolute Störsicherheit gegeben sein muß. Auch die Übertragung selbst im nichtgestörten Zustand muß so sicher sein, daß nur die Funktion, die der Chirurg über den Fußschalter anwählt, mit hoher Sicherheit geschaltet wird. Ein weiteres Problem ergibt sich, wenn möglicherweise in zwei benachbarten Operationssälen Fußschalter der erfindungsgemäßen Art verwendet werden, deren gegenseitige Störung ausgeschlossen sein muß. Eine diesbezüglich vorteilhafte Weiterbildung der Erfindung entnimmt man Anspruch 4 und 5.

Durch die Digitalisierung bzw. Kodierbarkeit der übertragenen Signale lassen sich Störeinflüsse ebenso wie gegenseitige Beeinflussungen benachbart betriebener Fußschalter vollständig ausschließen.

Besonders vorteilhaft ist es, wenn nach Anspruch 6 der Sendeteil direkt in den Fußschalter eingebaut ist. Aufgrund moderner elektronischer Bauelemente kann so ein Fußschalter geschaffen werden, der sich äußerlich von einem Fußschalter mit Leitungsübertragung praktisch nicht unterscheidet, jedoch eine drahtlose Übertragung der Schaltsignale zur Empfangsstation ermöglicht.

Um eine völlige Unabhängigkeit des erfindungsgemäß verwendeten Schalters von irgendwelchen äußeren Energiequellen zu gewährleisten, erfolgt der Betrieb bevorzugt nach Anspruch 7.

Um die Probleme mit einer bevorstehenden Entleerung der Batterien oder Akkumulatoren zu beherrschen, ist die Weiterbildung nach Anspruch 8 zweckmäßig.

Nachdem zwischen dem Sendeteil und der Empfangsstation keine körperliche Verbindung besteht, ist die Ausführungsform nach Anspruch 9 vorteilhaft, damit auf jeden Fall während des Nichtgebrauchs Sendeteil und Empfangsstation eine körperliche Einheit bilden.

Zur Wiederaufladung von in dem Sendeteil vorgesehenen Akkumulatoren soll nach Anspruch 12 ein Ladegerät vorgesehen sein.

Zum Zwecke der Stromversorgung des Ladegerätes ist es zweckmäßig, wenn die Empfangsstation nach Anspruch 11 mit dem Stromnetz verbindbar ist.

Eine weitgehend automatische Aufladung der Akkumulatoren in dem Sendeteil wird durch die Maßnahmen nach den Ansprüchen 12 bis 14 gewährleistet.

Nachdem die erfindungsgemäße Empfangsstation sehr klein dimensioniert sein kann, ist es zweckmäßig, nach Anspruch 15 eine gesonderte Ladestation für den Sendeteil vorzusehen, die an geeigneter Stelle und gegebenenfalls relativ weit entfernt von der Empfangsstation angeordnet werden kann. Die Ladestation soll eine Halterung für den Sendeteil aufweisen, so daß dieser bei Nichtgebrauch dort angebracht werden kann und während der Zeit des Nichtgebrauchs eine Aufladung der Akkumulatoren erfolgt.

Die Erfindung wird im folgenden beispielsweise anhand der Zeichnung beschrieben; in dieser zeigt:

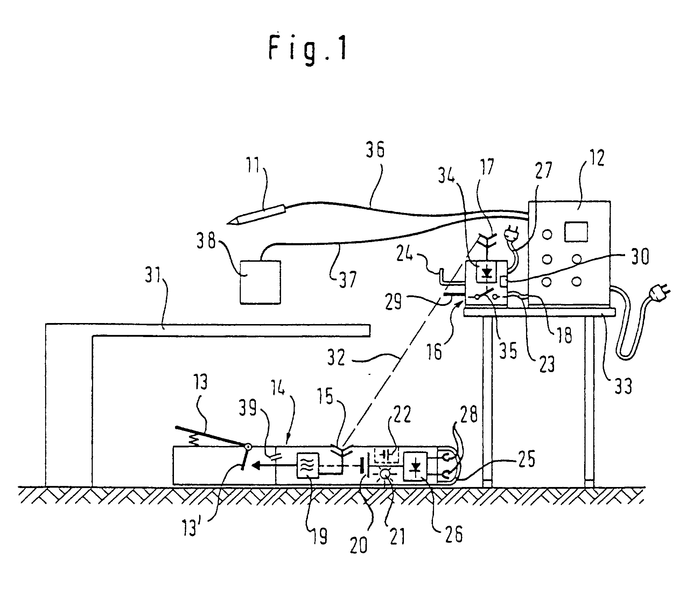

- Fig. 1: eine schematische Darstellung eines erfindungsgemäß geschalteten Hochfrequenz-Chirurgiegerätes in Anordnung an einem medizinischen Operationstisch.

Nach Fig. 1 ist neben einem in einem Operationssaal vorgesehenen Operationstisch 31 ein Beistelltisch 33 angeordnet, auf dem sich ein Hochfrequenz-Chirurgiegerät 12 befindet. Unterhalb des Operationstisches ist auf dem Boden ein Fußschalter 13 vorgesehen, der vom am Operationstisch 31 arbeitenden Chirurgen durch Niedertreten veranlaßt werden kann, einen Schaltkontaktpaar 13' elektrisch zu schließen oder zu öffnen.

Erfindungsgemäß ist der Fußschalter 13 Bestandteil eines Sendeteils 14, die eine an das Schaltkontaktpaar 13' angeschlossene Schaltsignal-Umwandlungsstufe 19 aufweist, in der beim Schließen des Schaltkontaktpaares 13' ein entsprechend digitalisiertes Hochfrequenzsignal erzeugt werden kann, welches über eine in dem Sendeteil 14 vorgesehene Sendeantenne 15 über einen gestrichelt angedeuteten Übertragungspfad 32 drahtlos zur Empfangsantenne 17 einer Empfangsstation 16 übertragen werden kann, die an geeigneter Stelle im Operationssaal untergebracht ist. Die elektrische Speisung der Schaltsignal-Umwandlungsstufe 19 in dem Sendeteil 14 erfolgt über einen Akkumulator 20, der von einem Ladegerät 26 aufgeladen werden kann. Weiter befindet sich innerhalb des Sendeteils 14 ein Reserveakkumulator 22, der bei Entladung des Hauptakkumulators 20 automatisch mit der Schaltsignal-Umwandlungsstufe 19 verbunden wird, um dessen elektrische Speisung zu übernehmen.

Eine insbesondere akustische Alarmanzeige 21 innerhalb des Sendeteils 14 spricht an, wenn der Hauptakkumulator 20 entladen ist und der Reserveakkumulator 22 die Speisung übernommen hat.

Vom Ladegerät 26 führen zur Außenseite des Sendeteils 14 zwei Ladeanschlüsse 28, über die das Ladegerät 26 mit elektrischer Energie versorgt werden kann.

Die Empfangsstation 16 weist einen an die Empfangsantenne 17 angeschlossenen Demodulator 34 auf, der das Empfangssignal demoduliert und dementsprechend einen in der Empfangsstation 16 vorgesehenen Schalter 35 betätigt. Der Schalter 35 führt auf diese Weise die gleichen Schaltvorgänge aus, wie das Schaltkontaktpaar 13' des Fußschalters 13.

Von der Empfangsstation 16, die über eine Netzanschlußleitung 27 in nicht dargestellter Weise mit dem Stromnetz verbunden ist, zweigt eine Steuerleitung 23 zu einem Speise- und Steuergerät in Form eines Hochfrequenz-Chirurgiegerätes 12 ab und ist mit dessen Fußschalter-Schalteingang 18 aufgrund eines entsprechend gestalteten Kupplungselementes verbunden. Der Schalter 35 der Empfangsstation 16 bewirkt im Hochfrequenz-Chirurgiegerät 12 die gleichen Schaltvorgänge, als wenn ein herkömmlicher Fußschalter 13 unmittelbar über eine Schaltleitung mit dem Schalteingang 18 verbunden ist.

Vom Hochfrequenz-Chirurgiegerät 12 erstrecken sich Hochfrequenzleitungen 36, 37 zu einem vom Chirurgen zu bedienendes hochfrequenz-chirurgisches Elektroinstrument 11 bzw. einer Neutralelektrode 38, die mit dem auf dem Operationstisch 31 befindlichen Patienten in elektrisch leitenden Kontakt gebracht wird.

In der Empfangsstation 16 befindet sich außerdem eine Signalerkennungsstufe 30, die auf dem Übertragungsweg 32 gestörte Übertragungssignale noch sicher zu erkennen gestattet.

An der Empfangsstation 16 befindet sich eine Halterung 24, an der mittels eines Bügels 25 der Fußschalter 13 während des Nichtgebrauchs aufgehängt werden kann. Hierbei kommen die Ladeanschlüsse 28 des Sendeteils 14 automatisch in elektrisch leitenden Kontakt mit Gegenanschlüssen 29 der Empfangsstation 16, die solange die Empfangsstation 16 über die Netzanschlußleitung 27 mit dem Stromnetz verbunden ist, über die Ladeanschlüsse 28 automatisch eine Aufladung der Akkumulatoren 20 und 22 in dem Sendeteil 14 gewährleisten. Bei erneutem Gebrauch des Fußschalters 13 ist somit automatisch dafür gesorgt, daß beide Akkumulatoren 20, 22 sich im voll aufgeladenen Zustand befinden.

Um für den Fall, daß sich beide Akkumulatoren 20, 22 während einer Operation entladen, den Operationsvorgang nicht unterbrechen zu müssen, kann erfindungsgemäß ein zweiter Fußschalter 13 mit identisch ausgebildetem Sendeteil 14 zur Verfügung gestellt werden. Auch ist es möglich, daß der Fußschalterteil und der Sendeteil 14 über eine in Fig. 1 schematisch angedeutete Trennstelle 39 miteinander verbunden sind, so daß im Falle einer Entladung der Akkumulatoren 20, 22 der Fußschalterteil an der Trennstelle 39 vom Sendeteil getrennt werden kann, worauf sofort ein zweiter zur Verfügung gehaltener Sendeteil dort angesteckt wird, wodurch der Fußschalter 13 erneut voll betriebsbereit ist.

Bei Nichtgebrauch muß der Sendeteil 14 nicht an der Empfangsstation 16 eingehängt werden, sondern kann an eine irgendwo an der Wand befestigte Ladestation angehängt werden. Diese Ausführung ist deswegen bevorzugt, weil die Empfangsstation 16 sehr klein dimensioniert werden kann und daher z.B. auch in das Hochfrequenz-Chirurgiegerät 12 eingebaut sein kann. Es ist auch denkbar, die Empfangsstation 16 durch einen Steckadapter mit dem Hochfrequenz-Chirurgiegerät 12 zu verbinden, was gegebenenfalls an der Eingangsbuchse für das Kabel eines konventionellen Fußschalters vorgenommen werden könnte, indem die Empfangsstation 16 ein Ausgangssignal wie ein konventioneller Fußschalter liefert. Von besonderer Bedeutung für die Erfindung ist es also, daß der Stecker eines konventionellen Fußschalters erhalten bleibt und lediglich statt mit einem konventionellen Fußschalter mit der Empfangsstation 16 verbunden ist, die an ihrem Ausgang den Schalter 35 aufweist. Diese Ausbildung ist deswegen von besonderer Bedeutung, weil bei einem etwaigen Ausfall des erfindungsgemäßen Sendeteils 14 sofort ein konventioneller Fußschalter an das Gerät 12 angesteckt werden kann.

### Bezugszeichenliste

- 11: Elektroinstrument
- 12: Speise- und Steuergerät
- 13: Fußschalter
- 13': Schaltkontaktpaar
- 14: Sendeteil
- 15: Sendeantenne
- 16: Empfangsstation
- 17: Empfangsantenne
- 18: Schalteingang
- 19: Schaltsignal-Umwandlungsstufe
- 20: Hauptakkumulator
- 21: Alarmanzeige
- 22: Reserveakkumulator
- 23: Leitung
- 24: Halterung
- 25: Bügel
- 26: Ladegerät
- 27: Netzanschlußleitung
- 28: Ladeanschluß
- 29: Gegenanschluß
- 30: Signalerkennungsstufe
- 31: Operationstisch
- 32: Übertragungspfad
- 33: Beistelltisch
- 34: Demodulator
- 35: Schalter
- 36: Hochfrequenzleitung
- 37: Hochfrequenzleitung
- 38: Neutralelektrode
- 39: Trennstelle

## Patentansprüche

1. Elektrisch betriebene medizinische Vorrichtung, wie elektrochirurgische Vorrichtung, mit einem von einer Person handhabbaren Elektroinstrument (11), das an ein elektrisch betriebenes Speise- und Steuergerät (12), wie ein Hochfrequenz-Chirurgiegerät, anschließbar bzw. angeschlossen ist, welches Speise- und Steuergerät (12) mit einem von der Person zu betätigenden Schalter (13) verbunden ist, mittels dessen über ein von ihm ausgelöstes Schaltsignal eine oder mehrere Funktionen des Elektroinstrumentes (11) im Speise- und Steuergerät (12) ein- und ausgeschaltet werden können, wobei der Schalter (13) einem Sendeteil (14) zugeordnet ist, das die Schaltsignale in drahtlos übertragbare Signale umwandelt und über ein Sendeelement (15) zu einem Empfangselement (17) einer Empfangsstation (16) sendet, welche an den Schalteingang (18) des Speiseund Steuergeräts (12) angeschlossen ist und ein dem Schaltsignal entsprechendes Steuersignal an das Speise- und Steuergerät (12) überträgt,
**dadurch gekennzeichnet,**
**daß** eine die Empfangsstation (16) mit dem Speise- und Steuergerät (12) verbindende Verbindung (23) an ihrem Ende einen Steckkontakt bzw. eine Steckbuchse aufweist, die der eines herkömmlichen Fußchalters entspricht, oder die Empfangsstation (16) mittels eines Steckkontaktes unmittelbar an den Eingang des Speise- und Steuergerätes (12) anschließbar ist, der für den Steckkontakt eines konventionellen Fußschalters vorgesehen ist.

2. Vorrichtung nach Anspruch 1,
**dadurch gekennzeichnet,**
**daß** die drahtlos übertragenen Signale Infrarot-, Ultraschall- oder Hochfrequenzsignale sind.

3. Vorrichtung nach Anspruch 1 oder 2,
**dadurch gekennzeichnet,**
**daß** der Schalter (13) ein Fußschalter ist.

4. Vorrichtung nach einem der vorhergehenden Ansprüche,
**dadurch gekennzeichnet,**
**daß** in dem Sendeteil (14) eine Schaltsignalumwandlungsstufe (19) vorgesehen ist, die die vom Schalter (13) erhaltenen Schaltsignale in ein drahtlos übertragbares, digitalisiertes Signal umwandelt.

5. Vorrichtung nach einem der vorhergehenden Ansprüche,
**dadurch gekennzeichnet,**
**daß** die drahtlos übertragenen Signale kodierbar bzw. kodiert sind und daß die zugeordnete Empfangsstation (16) eine entsprechende Signalerkennungsstufe (30) aufweist.

6. Vorrichtung nach einem der vorhergehenden Ansprüche,
**dadurch gekennzeichnet,**
**daß** der Schalter (13) Bestandteil des Sendeteils ist.

7. Vorrichtung nach einem der vorhergehenden Ansprüche,
**dadurch gekennzeichnet,**
**daß** das Sendeteil (14) mittels Batterie oder wiederaufladbarer Akkumulatoren (20, 22) betrieben ist.

8. Vorrichtung nach Anspruch 7,
**dadurch gekennzeichnet,**
**daß** in dem Sendeteil (14) eine Alarmanzeige (21) eingebaut ist, die rechtzeitig vor Entladung der Batterien oder Akkumulatoren (20, 22) anspricht und ein Alarmsignal abgibt.

9. Vorrichtung nach einem der vorhergehenden Ansprüche,
**dadurch gekennzeichnet,**
**daß** die Empfangsstation (16) eine Halterung (24) für das Sendeteil (14) während des Nichtgebrauchs aufweist.

10. Vorrichtung nach einem der Ansprüche 7 - 9,
**dadurch gekennzeichnet,**
**daß** das Sendeteil (14) und/oder die Empfangsstation (16) ein Ladegerät (26) für die Akkumulatoren (20, 22) des Sendeteils (14) aufweisen.

11. Vorrichtung nach einem der vorhergehenden Ansprüche,
**dadurch gekennzeichnet,**
**daß** die Empfangsstation (16) direkt über eine Netzanschlußleitung (27) oder über das Speise- und Steuergerät (12) an das Stromnetz anschließbar ist.

12. Vorrichtung nach einem der Ansprüche 7 - 11,
**dadurch gekennzeichnet,**
**daß** bei Halterung des Sendeteils (14) an der Empfangsstation (16) der bzw. die Akkumulatoren (20, 22) des Sendeteils (14) automatisch aufgeladen werden.

13. Vorrichtung nach Anspruch 12,
**dadurch gekennzeichnet,**
**daß** bei Halterung des Sendeteils (14) an der Empfangsstation (16) während des Nichtgebrauchs Ladeanschlüsse (28) an dem Sendeteil (14) mit Gegenanschlüssen (29) an der Empfangsstation (16) in elektrischen Kontakt kommen.

14. Vorrichtung nach Anspruch 12,
**dadurch gekennzeichnet,**
**daß** bei Halterung des Sendeteils (14) an der Empfangsstation (16) während des Nichtgebrauchs die Empfangsstation (16) zur Aufladung der Akkumulatoren (20, 22) mit einem Ladegerät (26) in dem Sendeteil (14) induktiv gekoppelt ist.

15. Vorrichtung nach Anspruch 7 oder 8,
**dadurch gekennzeichnet,**
**daß** die Ladestation für den Sendeteil (14) separat von der Empfangsstation (16) an geeigneter Stelle vorgesehen ist und das Sendeteil (14) bei Nichtgebrauch von der Ladestation gehalten wird, um die Aufladung des Akkumulators bzw. der Akkumulatoren (20, 22) vorzunehmen.

## Claims

1. Electrically operated medical apparatus, such as an electro-surgical apparatus comprising an electrical instrument (11) which can be handled by a person and which is connectable or connected to an electrically operated feed and control apparatus (12), such as a radio frequency surgical apparatus, which feed and control apparatus (12) is connected to a switch (13) to be actuated by the person, by means of which a switching signal initiated by him can switch on and off one or more functions of the electrical instrument (11) in the feed and control apparatus (12), the switch (13) being associated with a transmitter part (14) which converts the switching signals into signals which can be transmitted without wires and which are transmitted via a transmitting element (15) to a receiving element (17) of a receiving station (16), which is connected to the switching input (18) of the feed and control apparatus (12) and transmits a control signal corresponding to the switching signal to the feed and control apparatus (12),
**characterised in that**
a connection connecting the receiving station (16) to the feed and control apparatus (12) has a plug contact or a socket at its end, which corresponds to that of a customary foot switch, or the receiving station (16) can be connected by means of a plug contact directly to the input of the feed and control apparatus (12), which is provided for the plug contact of a conventional foot switch.

2. Apparatus in accordance with claim 1, **characterized in that** the signals transmitted free of wire are infrared, ultrasonic or radio frequency signals.

3. Apparatus in accordance with claim 1 or claim 2, **characterized in that** the switch (13) is a foot switch.

4. Apparatus in accordance with any one of the preceding claims, **characterized in that** a switching signal conversion stage (19) is provided in the transmitter part (14), which converts the switching signals received from the switch (13) into a digitized signal which can be transmitted without wire.

5. Apparatus in accordance with any one of the preceding claims, **characterized in that** signals transmitted without a wire can be coded or are coded and **in that** the associated receiving station (16) has a corresponding signal recognition stage (30).

6. Apparatus in accordance with any one of the preceding claims, **characterized in that** the switch (13) is a component of the transmitter part.

7. Apparatus in accordance with any one of the preceding claims, **characterized in that** the transmitter part (14) is operated by means of a battery or rechargeable accumulators (20, 22).

8. Apparatus in accordance with claim 7, **characterized in that** an alarm indicator (21) is built into the transmitter part (14) and responds and transmits an alarm signal in good time before discharge of the batteries or accumulators (20, 22).

9. Apparatus in accordance with any one of the preceding claims, **characterized in that** the receiving station (16) has a holder (24) for the transmitter part (14) when it is not in use.

10. Apparatus in accordance with any one of the claims 7 - 9, **characterized in that** the transmitter part (14) and/or the receiving station (16) has a charging apparatus (26) for the accumulators (20, 22) in the transmitter part (14)

11. Apparatus in accordance with any one of the preceding claims, **characterized in that** the receiving station (16) can be connected directly via a mains connection lead (27) or via the feed and control apparatus (12) to the power mains.

12. Apparatus in accordance with any one of the claims 7 - 11, **characterized in that** when the transmitter part (14) is held at the receiving station (16) the accumulator or accumulators (20, 22) of the transmitter part (14) are automatically charged up.

13. Apparatus in accordance with claim 12, **characterized in that** when the transmitter part (14) is held at the receiving station (16) during non-use, charging connections (28) on the transmitter part (14) come into electrical contact with counter-connections (29) at the receiving station (16).

14. Apparatus in accordance with claim 12, **characterized in that** when the transmitter part (14) is held at the receiving station (16) during non-use, the receiving station (16) is coupled inductively to a charging apparatus (26) in the transmitter part (14) for the charging up of the accumulators (20, 22).

15. Apparatus in accordance with claim 7 or 8, **characterized in that** the charging station for the transmitter part (14) is provided separately from the receiving station (16) at a suitable position, and the transmitter part (14) is held when not used by the charging station, in order to effect the charging up of the accumulator or of the accumulators (20, 22).

## Revendications

1. Appareil médical à fonctionnement électrique, comme un appareil électrochirurgical, comportant un instrument électrique (11) manipulable par une personne et branché ou susceptible d'être branché à un dispositif d'alimentation et de commande (12) à fonctionnement électrique, tel qu'un dispositif chirurgical à haute fréquence, ledit dispositif d'alimentation et de commande (12) étant connecté à un commutateur (13), actionné par la personne, au moyen duquel, via un signal de commutation déclenché par celui-ci, une ou plusieurs fonctions de l'instrument électrique (11) peuvent être mises en service ou arrêtées dans le dispositif d'alimentation et de commande (12), le commutateur (13) étant associé à une partie émettrice (14) qui convertit les signaux de commutation en signaux transmissibles sans fil et les envoie via un élément émetteur (15) à un élément récepteur (17) d'une station réceptrice (16) qui est branchée à l'entrée de commutation (18) du dispositif d'alimentation et de commande (12) et qui transmet un signal de commande correspondant au signal de commutation vers le dispositif d'alimentation et de commande (12),
**caractérisé en ce qu'**une connexion (23) connectant la station réceptrice (16) au dispositif d'alimentation et de commande (12) présente à son extrémité un contact mâle ou un contact femelle qui correspond à celui d'un interrupteur à pied habituel, ou **en ce que** la station réceptrice (16) est susceptible d'être branchée au moyen du contact mâle directement à l'entrée du dispositif d'alimentation ou de commande (12), laquelle est prévue pour le contact mâle d'un interrupteur à pied traditionnel.

2. Appareil selon la revendication 1, **caractérisé en ce que** les signaux transmissibles sans fil sont des signaux infrarouges, ultrasonores ou à haute fréquence.

3. Appareil selon l'une ou l'autre des revendications 1 et 2, **caractérisé en ce que** le commutateur (13) est un interrupteur à pied.

4. Appareil selon l'une des revendications précédentes, **caractérisé en ce que** dans la partie émettrice (14) est prévu un étage de conversion de signal de commutation (19) qui convertit les signaux de commutation reçus par le commutateur (13) en un signal numérisé transmissible sans fil.

5. Appareil selon l'une des revendications précédentes, **caractérisé en ce que** les signaux transmissibles sans fil sont codés ou susceptibles d'être codés, et **en ce que** la station émettrice associée (16) comprend un étage de reconnaissance de signaux correspondant (30).

6. Appareil selon l'une des revendications précédentes, **caractérisé en ce que** le commutateur (13) est un composant de la partie émettrice.

7. Appareil selon l'une des revendications précédentes, **caractérisé en ce que** la partie émettrice (14) est alimentée au moyen de piles ou d'accumulateurs rechargeables (20, 22).

8. Appareil selon la revendication 7, **caractérisé en ce que** dans la partie émettrice (14) est monté un affichage d'alerte (21) qui réagit à temps avant la décharge des piles ou des accumulateurs (20, 22) et qui émet un signal d'alerte.

9. Appareil selon l'une des revendications précédentes, **caractérisé en ce que** la station émettrice (16) comprend une monture (24) pour la partie émettrice (14) pendant la non-utilisation.

10. Appareil selon l'une des revendications 7 à 9, **caractérisé en ce que** la partie émettrice (14) et/ou la station réceptrice (16) comporte(nt) un chargeur (26) pour les accumulateurs (20, 22) de la partie émettrice (14).

11. Appareil selon l'une des revendications précédentes, **caractérisé en ce que** la station réceptrice (16) est susceptible d'être branchée au secteur directement via une ligne de branchement au secteur (27) ou via le dispositif d'alimentation et de commande (12).

12. Appareil selon l'une des revendications 7 à 11, **caractérisé en ce que** lors d'un rangement de la partie émettrice (14) sur la station réceptrice (16), le ou les accumulateurs (20, 22) de la partie émettrice (14) sont chargés automatiquement.

13. Appareil selon la revendication 12, **caractérisé en ce que** lors d'un rangement de la partie émettrice (14) sur la station réceptrice (16) pendant la non-utilisation, des bornes de chargement (28) sur la partie émettrice (14) viennent en contact électrique avec des bornes complémentaires (29) sur la station réceptrice (16).

14. Appareil selon la revendication 12, **caractérisé en ce que** lors d'un rangement de la partie émettrice (14) sur la station réceptrice (16) pendant la non-utilisation, la station réceptrice (16) est couplée par voie inductive à un chargeur (26) dans la partie émettrice (14) pour charger les accumulateurs (20, 22).

15. Appareil selon l'une ou l'autre des revendications 7 et 8, **caractérisé en ce que** la station de chargement pour la partie émettrice (14) est prévue de façon séparée de la station réceptrice (16) à un emplacement approprié, et **en ce que** la partie émettrice (14) est maintenue par la station de chargement en cas de non-utilisation, afin de procéder au chargement de l'accumulateur ou des accumulateurs (20, 22).
